# EUROPÄISCHE PATENTSCHRIFT

(11) **EP 3 212 251 B1**
(45) Veröffentlichungstag und Bekanntmachung des Hinweises auf die Patenterteilung: **30.03.2022**
(21) Anmeldenummer: 15787571.7
(22) Anmeldetag: 28.10.2015
(51) Int. Cl.: A61M 1/16

(54) **DIALYSEKONZENTRAT-HERSTELLUNGSANORDNUNG**
ARRANGEMENT FOR PRODUCING DIALYSIS CONCENTRATE
SYSTÈME DE PRÉPARATION D'UN CONCENTRÉ POUR DIALYSE

(30) Priorität: 30.10.2014 DE 202014105204 U
(43) Veröffentlichungstag der Anmeldung: 06.09.2017
(73) Patentinhaber: INTERMEDT Medizin & Technik GmbH, 26842 Ostrhauderfehn (DE)
(72) Erfinder: DUMSCHAT, Christoph, 26789 Leer (DE)
(74) Vertreter: terpatent Patentanwälte ter Smitten Eberlein-Van Hoof Rütten Daubert
(86) Internationale Anmeldenummer: PCT/EP2015/075014
(87) Internationale Veröffentlichungsnummer: WO 2016/066699

(56) Entgegenhaltungen:
- EP-A1- 0 491 981
- WO-A1-96/25214
- WO-A1-2008/082033
- DE-A1- 19 931 077
- DE-A1-102009 031 473
- DE-B3- 10 313 965
- US-A1- 2011 155 657

## Beschreibung

Die Erfindung bezieht sich auf eine Dialysekonzentrat-Herstellungsanordnung zur Herstellung einer Dialysekonzentratflüssigkeit durch Lösen eines Trockenkonzentrats in Wasser.

Für die Durchführung einer typischen Dialysebehandlung werden üblicherweise 150-200 l Dialyseflüssigkeit benötigt. Die Dialyseflüssigkeit wird in der Regel in dem Dialysegerät aus zwei Dialysekonzentratflüssigkeiten und Wasser gemischt, die beispielsweise in einem Verhältnis 1/35 miteinander vermischt werden. Für die Herstellung der Dialyseflüssigkeit werden eine basische Dialysekonzentratflüssigkeit und eine saure Dialysekonzentratflüssigkeit mit Wasser homogen vermischt.

Für die Durchführung einer typischen Dialysebehandlung wird bis zu 5 l saubere Dialysekonzentratflüssigkeit benötigt, wobei ca. 90 % hiervon wiederum Wasser ist. Aus DE 199 31 077 A1 und DE 103 13 965 B3 ist jeweils eine typische Dialysekonzentrat-Herstellungsanordnung bekannt, durch die zur Herstellung der Dialysekonzentratflüssigkeit ein Trockenkonzentrat mit Wasser homogen vermischt wird. Das Trockenkonzentrat wird in einem mobilen und mehrfach verwendbaren Wechselbehälter angeliefert, der Anschlüsse zum Einleiten bzw. Ableiten von Flüssigkeit aufweist. Die beiden Anschlüsse des Wechselbehälters werden am Einsatzort an eine stationäre Herstellungsanlage angeschlossen, die unter anderem einen sogenannten Vorlagebehälter und eine Förderpumpe stromabwärts eines Auslasses des Vorlagebehälters aufweist, die die Flüssigkeit von dem Vorlagebehälter in den Wechselbehälter pumpt. Hierdurch wird die Flüssigkeit in der Herstellungsanordnung zwischen dem Vorlagebehälter und dem Wechselbehälter kontinuierlich im Kreis gepumpt, bis das Dialysat-Trockenkonzentrat vollständig und homogen in dem gesamten Flüssigkeitsvolumen gelöst ist. Schließlich wird die auf diese Weise zu einer Dialysekonzentratflüssigkeit gemischte Flüssigkeit in dem Vorlagebehälter gesammelt, und anschließend über einen Entladungs-Abfluss und einen anschließenden Entladungspfad zur Herstellung der Dialyseflüssigkeit abgepumpt.

Für die fertiggestellte Dialysekonzentratflüssigkeit muss sichergestellt sein, dass hierin keine korposkulären Bestandteile enthalten sind, insbesondere kein ungelöstes Trockenkonzentrat, das lokal zu einer Inhomogenität der Konzentration der Bestandteile des Trockenkonzentrats in der Dialysekonzentratflüssigkeit führen würde. Gemäß DIN EN 13867 ist hierzu eine Feinfiltrierung mit einem Filter mit einer Porengröße von 1,2 µm oder kleiner vorzusehen. Ein derartiger Feinfilter wird im Stand der Technik im Bereich der Herstellungsanlage in einem Flusspfad angeordnet, insbesondere im Entladungspfad. Angesichts der nicht unerheblichen Menge an korposkulären Bestandteilen in einer Charge Dialysekonzentratflüssigkeit muss der anlagenseitige Feinfilter schon nach wenigen Dialysekonzentrat-Herstellungszyklen ausgetauscht werden, was für das bedienende Personal umständlich, zeitraubend und darüber hinaus relativ kostspielig ist.

Aus EP 0 491 981 A1 und DE 10 2009 031 473 A1 ist jeweils eine Dialysekonzentrat-Herstellungsanordnung offenbart, wobei der Filter fluidisch zwischen dem Wechselbehälter-Ablaufanschluss und dem Misch-Einlass des Vorlagenbehälters angeordnet ist. Die Umwälzpumpe ist fluidisch zwischen dem Misch-Auslass des Vorlagebehälters und dem Wechselbehälter angeordnet, sodass die Umwälzpumpe einen Überdruck aufbauen muss, um den Druckabfall über den Filter zu kompensieren.

Aufgabe der Erfindung vor diesem Hintergrund ist es, eine zuverlässige Dialysekonzentrat-Herstellungsanordnung zu schaffen.

Diese Aufgabe wird erfindungsgemäß gelöst mit einer Dialysekonzentrat-Herstellungsanordnung mit den Merkmalen des Anspruchs 1.

Die erfindungsgemäße Herstellungsanordnung weist, wie aus dem Stand der Technik bekannt, eine stationäre Herstellungsanlage und einen mobilen Wechselbehälter mit dem Trockenkonzentrat auf. Die Herstellungsanlage weist einen großvolumigen Vorlagebehälter mit einem Misch-Einlass und einem Misch-Auslass auf. Das Volumen des Vorlagebehälters entspricht in der Regel einem Vielfachen der für eine einzige Dialysebehandlung erforderlichen Dialysekonzentratflüssigkeit.

Der Wechselbehälter weist einen Ablaufanschluss auf, der mit dem Misch-Einlass des Vorlagebehälters fluidisch verbunden ist. Ferner weist der Wechselbehälter einen fluidischen Kombianschluss auf, der fluidisch mit dem Misch-Auslass des Vorlagebehälters verbunden ist. Vorzugsweise ist zwischen dem Misch-Auslass des Vorlagebehälters und dem Kombianschluss des Wechselbehälters eine Förderpumpe zum Pumpen der Flüssigkeit von dem Vorlagebehälter in den Wechselbehälter vorgesehen.

Gemäß der Erfindung ist dem Wechselbehälter-Ablaufanschluss ein Auslassfilter fluidisch zugeordnet, der als Feinfilter mit einer Porengröße von unter 1,5 µm, besonders bevorzugt von unter 1,2 µm ausgebildet ist. Der für die normgerechte Feinfilterung erforderliche Feinfilter ist also Teil des Wechselbehälters, der nach jedem Dialysekonzentrat-Herstellungszyklus ohnehin komplett gegen einen neuen und mit Trockenkonzentrat neu aufgefüllten Wechselbehälter ausgetauscht wird. Im Bereich der stationären Herstellungsanlage kann daher theoretisch auf einen Feinfilter mit einer normgerechten Porengröße vollständig verzichtet werden. Selbst wenn im Bereich der stationären Herstellungsanlage aber ein zweiter Feinfilter angeordnet ist, so muss dieser zweite Feinfilter bei jedem Herstellungszyklus nur noch einen Bruchteil der Feststoffe zurückhalten, muss also in der Praxis erst nach Dutzenden von Herstellungszyklen ausgetauscht werden. Der Aufwand für den Austausch des anlagenseitigen Feinfilters wird also, wenn dieser überhaupt noch vorgesehen ist, gegenüber dem Stand der Technik auf 10% oder weniger reduziert.

Andererseits ist der Aufwand für das Austauschen bzw. das Reinigen des wechselbehälterseitigen Feinfilters nicht besonders hoch, da der Wechselbehälter vor einer Neubefüllung mit Trockenkonzentrat ohnehin geöffnet, gereinigt, gegebenenfalls repariert und wieder mit Trockenkonzentrat befüllt werden muss. Der routinemäßige Austausch des wechselbehälterseitigen Feinfilters stellt dabei nur einen sehr geringen Mehraufwand dar.

In der Mischphase eines Herstellungszyklus wird die Flüssigkeit aus dem Misch-Auslass des Vorlagebehälters kommend zu dem Kombianschluss des Wechselbehälters gepumpt. Die in den Wechselbehälter einfließende Flüssigkeit vermischt sich mit dem in dem Wechselbehälter befindlichen Trockenkonzentrat bzw., bei mit Flüssigkeit gefüllten Wechselbehälter, mit der sich in den Wechselbehälter bereits befindenden Flüssigkeit, und wird anschließend durch den Auslassfilter in den Ablaufanschluss gepumpt. Die in der Herstellungsanordnung zirkulierende Flüssigkeit durchströmt also bei jedem Verlassen des Wechselbehälters den als Feinfilter ausgebildeten Auslassfilter. Der Auslassfilter ist also in dem zirkulären Mischpfad angeordnet, so dass die das gesamte Flüssigkeitsvolumen vielfach durch den Auslassfilter gefiltert wird, was das Filterergebnis und die Redundanz der Filtrierung erheblich verbessert.

In der sich an die Mischphase anschließenden Sammelphase wird der Wechselbehälter durch Abpumpen der Flüssigkeit durch den Kombianschluss geleert. Die in und an dem Auslassfilter anhaftenden Feststoffe bleiben an dem Auslassfilter praktisch vollständig haften, so dass auch das in der Sammelphase aus dem Wechselbehälter in den Vorlagebehälter gepumpte Flüssigkeitsvolumen die erforderliche Feststofffreiheit und Reinheit aufweist.

Der Wechselbehälter-Ablaufanschluss ist mit einer Saugpumpe fluidisch verbunden, die die Flüssigkeit von dem Wechselbehälter-Ablaufanschluss zu dem Misch-Einlass des Vorlagebehälters pumpt. Durch den als Feinfilter ausgebildeten Auslassfilter tritt über den Auslassfilter ein erheblicher Druckabfall auf. Ohne die Saugpumpe muss dieser Druckabfall ausschließlich durch eine entsprechende Druckpumpe vor dem Kombianschluss des Wechselbehälters kompensiert werden, so dass die Flüssigkeit in dem mobilen Wechselbehälter während der Mischphase unter einem erheblichen Überdruck steht. Um ein Platzen des Wechselbehälters sowie eine unerwünschte Flüssigkeitsleckage zu verhindern, muss erheblicher konstruktiver Aufwand betrieben werden. Durch die Anordnung der Saugpumpe fluidisch hinter dem Wechselbehälter-Ablaufanschluss kann der Fluiddruck innerhalb des Wechselbehälters erheblich reduziert werden, da die Saugpumpe den Druckabfall über dem als Feinfilter ausgebildeten Auslassfilter kompensieren oder überkompensieren kann. Auf diese Weise kann der Wechselbehälter während der Mischphase annähernd druckfrei betrieben und gehalten werden.

Vorzugsweise mündet der Kombianschluss des Wechselbehälters in einer Fluidöffnung in Bodennähe des Wechselbehälters. Der Kombianschluss kann beispielsweise außen an dem Wechselbehälter-Deckel vorgesehen sein und in ein vertikales Steigrohr innerhalb des Wechselbehälters münden, dessen Fluidöffnung in Bodennähe des Wechselbehälters innerhalb des Wechselbehälters mündet, beispielsweise tangential orientiert. In der Mischphase eines Herstellungszyklus wird die Flüssigkeit also in Bodennähe und mit einer tangentialen Komponente in den Wechselbehälter eingeleitet, so dass auf diese Weise eine schnelle und homogene Auflösung des Trockenkonzentrats in der in den Wechselbehälter einfließenden Flüssigkeit gewährleistet ist. In der Sammelphase wird durch die Kombianschluss-Fluidöffnung in Bodennähe sichergestellt, dass der Wechselbehälter vollständig entleert werden kann.

Alternativ zu einem völligen Verzicht auf einen herstellungsanlagenseitigen Feinfilter kann in einer besonders bevorzugten Ausführung ein als Feinfilter ausgebildeter Redundanzfilter vorgesehen sein. Dieser kann besonders bevorzugt hinter einem Entladungs-Auslass des Vorlagebehälters angeordnet sein. Der Entladungs-Auslass ist ein Vorlagebehälter-Auslass, über den nach der Sammelphase die fertige Dialysekonzentratflüssigkeit zur weiteren Nutzung abgelassen bzw. abgepumpt wird. Der Redundanzfilter ist zwar für eine normgerechte Herstellung der Dialysekonzentratflüssigkeit in nicht unbedingt erforderlich, sorgt jedoch für eine zusätzliche Filtrier-Sicherheit. Da der Redundanzfilter vorliegend nicht im Mischkreis, sondern im Entladungspfad angeordnet ist, sind die Feststoff-Mengen, die bei jedem Herstellungszyklus durch den Redundanzfilter zurückgehalten werden müssen, sehr gering. Ein Austausch des Redundanzfilters ist daher in der Regel erst nach 20-40 Herstellungszyklen erforderlich.

Gemäß einer bevorzugten Ausgestaltung ist dem Wechselbehälter-Kombianschluss ein Kombianschluss-Filter fluidisch zugeordnet, dessen Porengröße über 1,5 µm liegt. Durch den Kombianschluss-Filter ist also ein Grobfilter, durch den die größeren korposkulären Bestandteile während der Mischphase der in den Wechselbehälter einlaufenden Flüssigkeit zurückgehalten werden. Hierdurch wird sichergestellt, dass der als Feinfilter ausgebildeter Auslassfilter nur mit feinen Feststoffen belastet wird.

Vorzugsweise weist der Auslassfilter eine Stützstruktur auf, die eine separate Filterstruktur trägt. Zwar könnte für den Auslassfilter grundsätzlich auf eine steife und feste Stützstruktur verzichtet werden, jedoch können in dem Wechselbehälter erhebliche Flüssigkeitsströmungen auftreten. Durch die separate steife Stützstruktur weist der Auslassfilter eine ausreichende Steifheit und Festigkeit auf, um zuverlässig eine Deformation, eine Beschädigung oder gar ein Abbrechen des Auslassfilters durch die auftretenden Strömungskräfte auszuschließen.

Vorzugsweise wird die Stützstruktur von einem hohlzylindrischen Stützkörper mit lateralen Öffnungen in der seitlichen Stützkörperwand gebildet, Der hohlzylindrische Stützkörper ist bevorzugt vertikal angeordnet, so dass die Flüssigkeit durch die lateralen Öffnungen bevorzugt horizontal einströmt. Besonders bevorzugt ist der hohlzylindrische Stützkörper becherartig ausgebildet und an seinem unteren Längsende geschlossen, so dass die gesamte Flüssigkeit durch die lateralen Stützkörper-Öffnungen einströmt.

Gemäß einer bevorzugten Ausgestaltung weist der Auslassfilter eine Filterstruktur mit einem Filterkörper auf, dessen Filterstärke an jeder Stelle mindestens 8,0 mm beträgt. Ferner ist besonders bevorzugt vorgesehen, dass der Filterkörper hohlzylindrisch, besonders bevorzugt becherförmig, ausgebildet ist und eine axiale Länge von mindestens 20 cm aufweist. Hierdurch wird trotz des hohen spezifischen Flusswiderstands des Feinfilters ein relativ geringer absoluter Flusswiderstand realisiert. Hierdurch wiederum kann die Pumpleistung entsprechend reduziert werden bzw. der Überdruck innerhalb des Wechselbehälters entsprechend klein gehalten werden.

Vorzugsweise weist der hohlzylindrische Stützkörper des Auslassfilters an seinem oberen Ende einen Außengewinde-Abschnitt mit einem Außengewinde auf, der bzw. das in ein korrespondierendes Innengewinde des Behälterdeckels des Wechselbehälters eingeschraubt ist. Der Stützkörper weist also eine Doppelfunktion auf, denn er bildet zum Einen die mechanische Versteifung für den Filterkörper und weist zum Anderen einen Gewindeabschnitt auf, wodurch der Auslassfilter auf einfache Weise schraubend entfernt und appliziert werden kann.

Im Folgenden wird ein Ausführungsbeispiel der Erfindung anhand der Zeichnungen näher erläutert. Es zeigen:
Figur 1 eine schematische Darstellung einer Dialysekonzentrat-Herstellungsanordnung mit einer stationären Herstellungsanlage und einem mobilen Wechselbehälter, zur Herstellung einer Dialysekonzentratflüssigkeit durch Lösen eines Trockenkonzentrats in Wasser, und
Figur 2 eine Schnittdarstellung eines Auslassfilters, der an einem Ablaufanschluss des Wechselbehälters angeordnet ist.

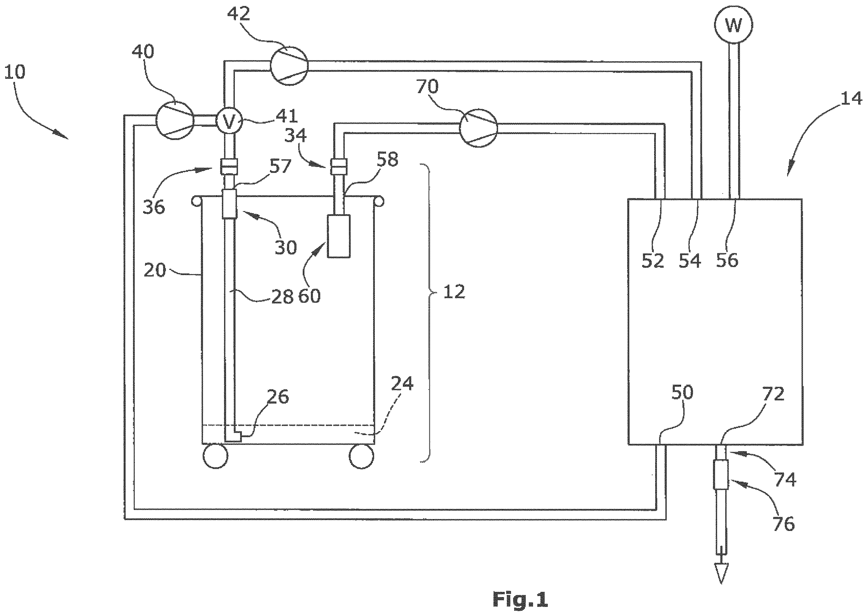

Die Figur 1 zeigt schematisch eine Dialysekonzentrat-Herstellungsanordnung 10 zur Herstellung einer Dialysekonzentratflüssigkeit, beispielsweise einer sauren Dialysekonzentratflüssigkeit, durch Lösen eines Trockenkonzentrats in Wasser. Die Herstellungsanordnung 10 besteht aus einem mobilen Wechselbehälter 12 mit einem Trockenkonzentrat 24 und aus einer stationären Herstellungsanlage, die mehrere Komponenten aufweist. Die Herstellungsanordnung 10 ist vorliegend nicht notwendigerweise vollständig dargestellt, sondern nur mit den Komponenten, die für die Beschreibung der Erfindung erforderlich sind.

Der mobile Wechselbehälter 12 ist tonnenartig ausgebildet und fluidisch geschlossen. Das Gehäuse des Wechselbehälters 12 besteht im wesentlichen aus einem becherförmigen Behälterkörper 20 und einem die obenliegende Becheröffnung abschließenden Behälterdeckel 22. Das auf diese Weise gebildete Wechselbehälter-Gehäuse ist fluidisch geschlossen. Der Wechselbehälter 12 kann Räder für seinen Transport aufweisen.

Der Wechselbehälter 12 weist an seinem Behälterdeckel 22 zwei Fluidikanschlüsse auf, nämlich einen bidirektionalen Kombianschluss 57 und einen unidirektionalen Ablaufanschluss 58. Dem Kombianschluss 57 und dem Ablaufanschluss 58 ist jeweils eine fluidische Leitungskupplung 36,34 zugeordnet, die jeweils als Schnellkupplung ausgebildet ist und einen schnellen fluidischen Anschluss oder aber eine schnelle fluidische Trennung des Wechselbehälters 12 an die bzw. von der stationären Herstellungsanlage erlaubt.

Der Kombianschluss 57 mündet in dem Wechselbehälter 12 über einen Kombianschluss-Filter 30 fluidisch in ein vertikales Steigrohr 28, an dessen unterem Ende eine tangentiale Fluidöffnung 26 vorgesehen ist, durch die die ausströmende Flüssigkeit in zirkulärer bzw. tangentialer Richtung in den Behälterkörper 20 einströmt, und hierbei gegebenenfalls das Trockenkonzentrat 24 von dem Behälterkörper 20 löst und mitreißt. Der Kombianschluss-Filter 30 weist eine Porengröße von über 1,5 µm auf.

Dem Ablaufanschluss 58 fluidisch zugeordnet ist innenseitig des Wechselbehälters 12 und unmittelbar von dem Behälterdeckel 22 vertikal nach unten abragend ein Auslassfilter 60 vorgesehen, der als Feinfilter mit einer Porengröße von unter 1,5 µm, beispielsweise mit einer Porengröße von nominell 1,1 µm ausgebildet ist.

Die Herstellungsanlage weist einen Vorlagebehälter 14 auf, der über mehrere Einlässe 52,54,56 verfügt, durch die ggf. Flüssigkeit in den Vorlagebehälter 14 einfließen kann, nämlich einen Misch-Einlass 52, einen Sammel-Einlass 54 und einen Füll-Einlass 56. Ferner weist der Vorlagebehälter 14 zwei Auslässe 50,72 auf, durch die ggf. Flüssigkeit aus dem Vorlagebehälter abfließen kann, nämlich einen Misch-Auslass 50 und einen Entladungs-Auslass 72. Der Vorlagebehälter 14 hat ein Volumen, das erheblich über dem Volumen des Wechselbehälters 12 liegt.

Der Misch-Einlass 52 des Vorlagebehälters 14 ist über eine entsprechende Leitung, in deren Verlauf eine Saugpumpe 70 angeordnet ist, mit der Leitungskupplung 34 des Ablaufanschlusses 58 fluidisch verbunden. Die Saugpumpe 70 pumpt in Richtung des Misch-Einlasses 52. Der Füll-Einlass 56 ist mit einer Wasserquelle W verbunden, durch die der Vorlagebehälter 14 zu Beginn eines Herstellungszyklus' mit einer entsprechenden Menge Wasser befüllt werden kann. Der Sammel-Einlass 54 ist fluidisch über eine entsprechende Leitung, in deren Verlauf eine Sammelpumpe 42 angeordnet ist, mit einem 3-Wege-Ventil 41 verbunden, dessen zweiter Zweig mit der Leitungskupplung 36 des Kombianschlusses 57 fluidisch verbunden ist. Der dritte Zweig des 3-Wege-Ventils 41 ist über eine Leitung, in deren Verlauf eine Mischpumpe 40 angeordnet ist, mit dem Misch-Auslass 50 des Vorlagebehälters 14 fluidisch verbunden. Die Sammelpumpe 42 pumpt in Richtung Vorlagebehälter 14. Die Mischpumpe 40 pumpt in Richtung 3-Wege-Ventil 41.

Der Entladungs-Auslass 72 des Vorlagebehälters 14 mündet in einen Entladungspfad 74, in dessen fluidischem Verlauf ein Redundanzfilter 76 angeordnet ist, der eine Porengröße von unter 1,2 µm aufweist.

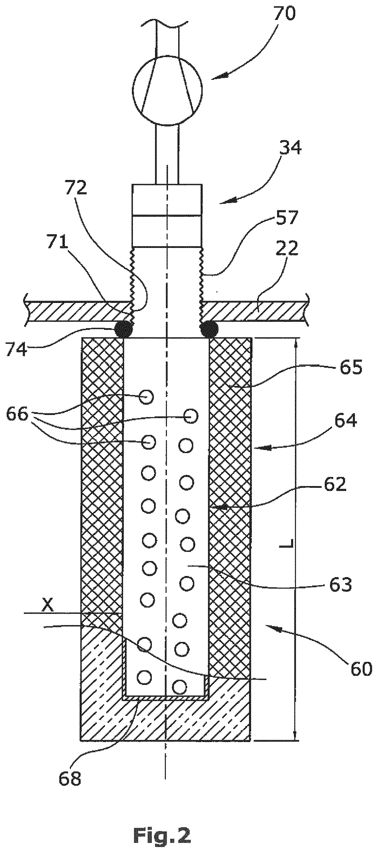

Der Auslassfilter 60 ist in der Figur 2 vergrößert und in Längsrichtung teilweise geschnitten dargestellt. Der Auslassfilter 60 weist als Stützstruktur 62 einen zylindrischen und becherförmigen Stützkörper 63 auf, der vertikal angeordnet ist und an seinem unteren Längsende einen geschlossenen Becherboden 68 aufweist. In der zylindrischen Seitenwand des Stützkörpers 63 ist eine Vielzahl von lateralen Fluidik-Öffnungen 66 angeordnet. Die Stützstruktur 62 stützt und hält eine Filterstruktur 64, die von einem ebenfalls becherförmigen Filterkörper 65 gebildet wird, der eine vertikale Länge L von ungefähr 25 cm aufweist. Der Filterkörper 65 besteht beispielsweise aus einem Polypropylen-Filtermaterial. Der Filterkörper 65 weist an jeder Stelle eine Filterstärke X von 10,0 mm auf, so dass der fluidische Weg der Flüssigkeit von außen durch den Filterkörper 65 in den Stützkörper 63 stets mindestens 10,0 mm beträgt.

An dem oberen Längsende weist die Stützstruktur 62 ein Außengewinde 71 auf, das in ein entsprechendes Innengewinde 72 des Behälterdeckels 22 eingeschraubt ist. Innenseitig ist zwischen dem Öffnungsrand des Behälterdeckels 22 und der Stützstruktur 62 ein Dichtring 74 angeordnet, durch den eine fluiddichte Abdichtung sichergestellt wird, so dass Fluid nur durch den Innenraum der Stützstruktur 62 aus dem Wechselbehälter 12 abfließen kann.

Ein Herstellungszyklus läuft wie folgt ab:
Zunächst wird der neu mit dem Trockensubstrat 24 befüllte Wechselbehälter 12 über die beiden Leitungskupplungen 34,36 fluidisch mit der Herstellungsanlage verbunden. Der Wechselbehälter 12 weist einen neuen oder aber einen ausgespülten Auslassfilter 60 auf. Nachdem der Start des Herstellungszyklus' ausgelöst ist, löst eine nicht dargestellte Anlagensteuerung zunächst das Auffüllen des Vorlagebehälters 14 mit Wasser aus der Wasserquelle W aus, so dass der Vorlagebehälter 14 mit einer Sollmenge Wasser gefüllt wird. Alle Pumpen 40,42,70 sind nicht in Betrieb und das 3-Wege-Ventil 41 ist geschlossen. Sobald der Vorlagebehälter 14 mit der Sollmenge an Wasser gefüllt ist, beginnt die Mischphase.

In der Mischphase wird die Mischpumpe 40 aktiviert und das 3-Wege-Ventil 41 so geschaltet, dass die von der Mischpumpe 40 kommende Flüssigkeit über den Kombianschluss 57 in den Wechselbehälter 12 eingeleitet wird. Die einströmende Flüssigkeit strömt tangential aus der Steigrohr-Fluidöffnung 26 aus, so dass hierdurch das pulverartige Trockenkonzentrat 24 von dem Flüssigkeitsstrom mitgerissen und in der Flüssigkeit gelöst wird. Der Flüssigkeitspegel in dem Wechselbehälter 12 steigt schließlich soweit an, dass der Wechselbehälter 12 vollständig mit Flüssigkeit gefüllt ist.

Spätestens zu diesem Zeitpunkt nimmt die Saugpumpe 70 ihren Betrieb auf, und pumpt die Flüssigkeit aus dem Wechselbehälter 12 zu dem Vorlagebehälter 14. Während der Mischphase wird auf diese Weise die gesamte systemische Flüssigkeit zwischen dem Vorlagebehälter 14 und dem Wechselbehälter 12 und wieder zurück im Kreis gepumpt, bis über entsprechende Sensoren eine ausreichende chemische Homogenität des gesamten systemischen Flüssigkeitsvolumens festgestellt wird. Da das systemische Flüssigkeitsvolumen hierbei den als Feinfilter ausgebildeten Auslassfilter 60 vielfach passieren muss, werden alle festen Bestandteile, beispielsweise ungelöstes Trockenkonzentrat, von dem Auslassfilter 60 zurückgehalten.

Sobald die Mischphase beendet ist, wird das 3-Wege-Ventil 41 derart umgeschaltet, dass durch die nunmehr aktivierte Sammelpumpe 42 die Flüssigkeit aus dem Wechselbehälter 12 über den Sammel-Einlass 54 in den Vorlagebehälter 14 vollständig abgepumpt wird. Sobald das gesamte Flüssigkeitsvolumen in dem Vorlagebehälter 14 versammelt ist, werden alle dargestellten Pumpen 40,42,70 stillgesetzt und Ventile geschlossen, so dass die Flüssigkeit, die nunmehr eine homogene Dialysekonzentratflüssigkeit darstellt, über den Entladungs-Auslass 72 und den Redundanzfilter 76 in den anschließenden Entladungspfad 74 zur weiteren Verwendung abgepumpt werden kann. Hierbei wird die fertige Dialysekonzentratflüssigkeit in dem als Feinfilter ausgebildeten Redundanzfilter 76 erneut normgerecht gefiltert.

## Patentansprüche

1. Dialysekonzentrat-Herstellungsanordnung (10) zur Herstellung einer Dialysekonzentratflüssigkeit durch Lösen eines Trockenkonzentrats (24) in Wasser, mit
einer stationären Herstellungsanlage, die einen Vorlagebehälter (14) mit einem Misch-Einlass (52) und einem Misch-Auslass (50) aufweist, und
einem mobilen Wechselbehälter (12) mit dem Trockenkonzentrat (24), wobei der Wechselbehälter (12) einen Ablaufanschluss (58) aufweist, der mit dem Misch-Einlass (52) des Vorlagebehälters (14) über eine erste Leitung fluidisch verbunden ist und einen Kombianschluss (57) aufweist, der mit dem Misch-Auslass (50) des Vorlagebehälters (14) über eine entsprechende zweite Leitung fluidisch verbunden ist, wobei
dem Wechselbehälter-Ablaufanschluss (58) ein Auslassfilter (60) fluidisch zugeordnet ist, der als Feinfilter mit einer Porengröße von unter 1,5 µm ausgebildet ist, und
**gekennzeichnet durch**
eine Saugpumpe (70) die die Flüssigkeit von dem Wechselbehälter-Ablaufausschluss (58) zu dem Misch-Einlass (52) pumpt und die in der ersten Leitung flussabwärts des Auslassfilters angeordnet ist und fluidisch mit dem Wechselbehälter-Ablaufanschluss (58) verbunden ist.

2. Dialysekonzentrat-Herstellungsanordnung (10) nach Anspruch 1, wobei der Kombianschluss (57) in einer Fluidöffnung (26) in Bodennähe innerhalb des Wechselbehälters (12) mündet.

3. Dialysekonzentrat-Herstellungsanordnung (10) nach einem der vorangegangenen Ansprüche, wobei der Vorlagebehälter (14) einen Entladungs-Auslass (72) aufweist, der in einen fluidischen Entladungspfad (74) mündet, in dessen fluidischem Verlauf ein Redundanzfilter (76) angeordnet ist.

4. Dialysekonzentrat-Herstellungsanordnung (10) nach Anspruch 3, wobei der Redundanzfilter (76) ein Feinfilter mit einer Porengröße von unter 1,5 µm ist.

5. Dialysekonzentrat-Herstellungsanordnung (10) nach einem der vorangegangenen Ansprüche, wobei dem Wechselbehälter-Kombianschluss (57) ein Kombianschluss-Filter (30) fluidisch zugeordnet ist, dessen Porengröße über 1,5 µm liegt.

6. Dialysekonzentrat-Herstellungsanordnung (10) nach einem der vorangegangenen Ansprüche, wobei der Auslassfilter (60) eine Stützstruktur (62) aufweist, die eine separate Filterstruktur (64) trägt.

7. Dialysekonzentrat-Herstellungsanordnung (10) nach Anspruch 6, wobei die Stützstruktur (62) von einem hohlzylindrischen Stützkörper (63) mit lateralen Öffnungen (66) gebildet wird.

8. Dialysekonzentrat-Herstellungsanordnung (10) nach einem der vorangegangenen Ansprüche, wobei der Auslassfilter (60) eine Filterstruktur (64) mit einem Filterkörper (65) aufweist, dessen Filterstärke (X) an jeder Stelle mindestens 8,0 mm beträgt.

9. Dialysekonzentrat-Herstellungsanordnung (10) nach Anspruch 8, wobei der Filterkörper (65) hohlzylindrisch ausgebildet ist und eine axiale Länge L von mindestens 20 cm aufweist.

10. Dialysekonzentrat-Herstellungsanordnung nach Anspruch 7, wobei der hohlzylindrische Stützkörper (63) des Auslassfilters (60) an seinem oberen Ende einen Außengewinde-Abschnitt mit einem Außengewinde (41) aufweist, der in ein korrespondierendes Innengewinde (20) eines Behälterdeckels (22) des Wechselbehälters (12) eingeschraubt ist.

## Claims

1. Dialysis concentrate preparation assembly (10) for preparing a dialysis concentrate liquid by dissolving a dry concentrate (24) in water, comprising
a stationary manufacturing plant comprising a storage tank (14) with a mixing inlet (52) and a mixing outlet (50), and
a mobile exchangeable container (12) with the dry concentrate (24), wherein the exchangeable container (12) comprises a discharge connection (58) which is fluidically connected to the mixing inlet (52) of the receiving container (14) via a first line and comprises a combined connection (57) which is fluidically connected to the mixing outlet (50) of the receiving container (14) via a corresponding second line, wherein
an outlet filter (60) is fluidically associated with the exchangeable container discharge connection (58), which filter is configured as a fine filter with a pore size of less than 1.5 µm, and
**characterised by**
a suction pump (70) which pumps the liquid from the exchangeable container discharge connection (58) to the mixing inlet (52) and which is arranged in the first line downstream of the outlet filter and is fluidically connected to the exchangeable container discharge connection (58).

2. Dialysis concentrate preparation assembly (10) according to claim 1, wherein said combined connection (57) leads into a fluid port (26) near the bottom within said exchangeable container (12).

3. Dialysis concentrate preparation assembly (10) according to any one of the preceding claims, wherein the storage container (14) comprises a discharge outlet (72) which leads into a fluidic discharge path (74), in the fluidic course of which a redundancy filter (76) is arranged.

4. Dialysis concentrate preparation assembly (10) according to claim 3, wherein the redundancy filter (76) is a fine filter with a pore size of less than 1.5 µm.

5. Dialysis concentrate preparation assembly (10) according to any one of the preceding claims, wherein the exchangeable container combined connection (57) has fluidically associated therewith a combined connection filter (30) having a pore size greater than 1.5 µm.

6. Dialysis concentrate preparation assembly (10) according to any one of the preceding claims, wherein the outlet filter (60) comprises a support structure (62) carrying a separate filter structure (64).

7. Dialysis concentrate preparation assembly (10) according to claim 6, wherein said support structure (62) is defined by a hollow cylindrical support body (63) having lateral openings (66).

8. Dialysis concentrate preparation assembly (10) according to any one of the preceding claims, wherein the outlet filter (60) comprises a filter structure (64) with a filter body (65), the filter thickness (X) of which is at least 8.0 mm at any point.

9. Dialysis concentrate preparation assembly (10) according to claim 8, wherein the filter body (65) is configured to be hollow cylindrical and comprises an axial length L of at least 20 cm.

10. Dialysis concentrate preparation assembly according to claim 7, wherein the hollow cylindrical support body (63) of the outlet filter (60) comprises at its upper end an externally threaded section with an external thread (41), which is screwed into a corresponding internal thread (20) of a container lid (22) of the exchangeable container (12).

## Revendications

1. Ensemble de préparation de concentré de dialyse (10) pour préparer un liquide de concentré de dialyse en dissolvant un concentré sec (24) dans de l'eau, comprenant
une installation de fabrication stationnaire comprenant un réservoir de dépôt (14) avec une entrée de mélange (52) et une sortie de mélange (50), et
un récipient échangeable mobile (12) avec le concentré sec (24), le récipient échangeable (12) comprenant une connexion de décharge (58) qui est reliée fluidiquement à l'entrée de mélange (52) du réservoir de dépôt (14) par une première conduite et comprenant une connexion combinée (57) qui est reliée fluidiquement à la sortie de mélange (50) du réservoir de dépôt (14) par une deuxième conduite correspondante, dans lequel
un filtre de sortie (60) est associé fluidiquement à la connexion de décharge du réservoir échangeable (58), lequel filtre est configuré comme un filtre fin avec une taille de pores inférieure à 1,5 µm, et **caractérisé par**
une pompe d'aspiration (70) qui pompe le liquide de la tubulure de sortie du réservoir interchangeable (58) vers l'entrée de mélange (52) et qui est disposée dans la première conduite en aval du filtre de sortie et est en liaison fluidique avec la tubulure de sortie du réservoir interchangeable (58).

2. Ensemble de préparation de concentré de dialyse (10) selon la revendication 1, dans lequel ladite connexion combinée (57) mène à un port de fluide (26) près du fond dans ledit récipient échangeable (12).

3. Ensemble de préparation de concentré de dialyse (10) selon l'une quelconque des revendications précédentes, dans lequel le réservoir de dépôt (14) comprend une sortie de décharge (72) qui mène dans un passage de décharge fluidique (74), dans le passage fluidique duquel un filtre de redondance (76) est disposé.

4. Ensemble de préparation de concentré de dialyse (10) selon la revendication 3, dans lequel le filtre de redondance (76) est un filtre fin dont la taille des pores est inférieure à 1,5 µm.

5. Ensemble de préparation de concentré de dialyse (10) selon l'une quelconque des revendications précédentes, dans lequel la connexion combinée (57) du réservoir échangeable est associée fluidiquement à un filtre de connexion combinée (30) ayant une taille de pore supérieure à 1,5 µm.

6. Ensemble de préparation de concentré de dialyse (10) selon l'une quelconque des revendications précédentes, dans lequel le filtre de sortie (60) comprend une structure de support (62) portant une structure de filtre séparée (64).

7. Ensemble de préparation de concentré de dialyse (10) selon la revendication 6, dans lequel ladite structure de support (62) est définie par un corps de support cylindrique creux (63) ayant des ouvertures latérales (66).

8. Ensemble de préparation de concentré de dialyse (10) selon l'une quelconque des revendications précédentes, dans lequel le filtre de sortie (60) comprend une structure de filtre (64) avec un corps de filtre (65), dont l'épaisseur de filtre (X) est d'au moins 8,0 mm en tout point.

9. Ensemble de préparation de concentré de dialyse (10) selon la revendication 8, dans lequel le corps de filtre (65) est configuré pour être cylindrique creux et comprend une longueur axiale L d'au moins 20 cm.

10. Ensemble de préparation de concentré de dialyse selon la revendication 7, dans lequel le corps de support cylindrique creux (63) du filtre de sortie (60) comprend à son extrémité supérieure une section filetée extérieurement avec un filet externe (41), qui est vissé dans un filet interne correspondant (20) d'un couvercle de réservoir (22) du réservoir échangeable (12).
